# EUROPEAN PATENT APPLICATION

(11) **EP 4 410 353 A1**
(43) Date of publication of application: **07.08.2024**
(21) Application number: 21959293.8
(22) Date of filing: 29.09.2021
(51) Int. Cl.: A61M 25/09

(54) **GUIDE WIRE**

(71) Applicant: Asahi Intecc Co., Ltd., Seto-shi, Aichi 489-0071 (JP)
(72) Inventor: MAEDA, Aoi, Seto-shi, Aichi 489-0071 (JP); TSUGE, Kenta, Seto-shi, Aichi 489-0071 (JP); YAMAGUCHI, Reo, Seto-shi, Aichi 489-0071 (JP)
(74) Representative: Winter, Brandl - Partnerschaft mbB
(86) International application number: PCT/JP2021/035773
(87) International publication number: WO 2023/053245

(57) **Abstract**

This guide wire comprises a core shaft. A main body part of the guide wire, which is a portion 350 mm or more and 750 mm or less from a distal end of the core shaft, is made of nickel-titanium alloy, and an outer diameter of the main body part is 0.58 mm or more and 0.73 mm or less.

## Description

### TECHNICAL FIELD

The disclosed embodiments relate to a guide wire.

### BACKGROUND ART

Guide wires are conventionally known as medical instruments that are used by being inserted percutaneously into a blood vessel to treat a constricted part occurring in the blood vessel. As such a guide wire, Patent Literature 1 describes a guide wire used in the treatment of lower limb blood vessels.

### CITATION LIST

### Patent Literature

Patent Literature 1: JP 2016-174645 A

### SUMMARY OF INVENTION

### TECHNICAL PROBLEM

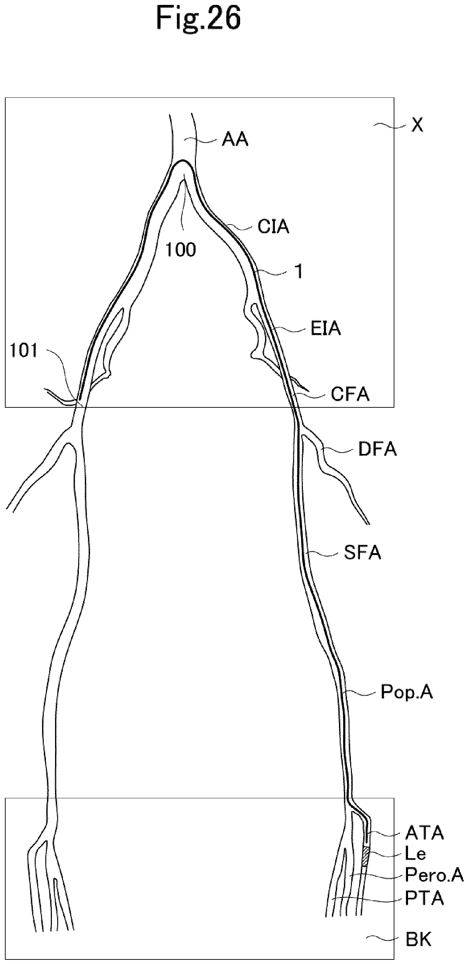

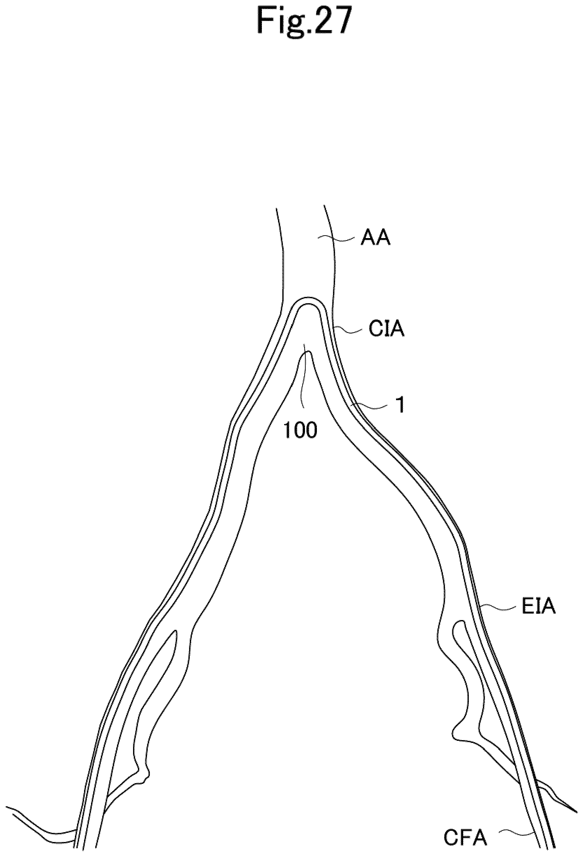

A technique called the crossover method is adopted as a method for treating a constricted part of a lower limb blood vessel using a guide wire. Fig. 26 is an explanatory diagram illustrating the lower limb blood vessels of a human body. Fig. 27 is an explanatory diagram showing enlarged the X region in Fig. 26. The crossover method is a method in which a guide wire 1 is inserted into a blood vessel from a punctured portion 101 near the femoral region of the leg that is not the leg having a constricted part Le, and then passed through the common iliac artery (hereinafter, referred to as CIA) and advanced to the leg having the constricted part Le until reaching the constricted part Le. Fig. 26 illustrates a state in which the guide wire 1 is inserted into the lower limb blood vessels of a human body. Here, it is assumed that the constricted part Le has occurred in the anterior tibial artery (hereinafter, referred to as ATA), which is included in the below-knee region (hereinafter, referred to as BK). The guide wire 1 is firstly percutaneously inserted into a blood vessel inside the body from the punctured portion 101 near the femoral region of the leg that is not the leg in which the constricted part Le has occurred. The guide wire 1 is advanced through the CIA toward the abdominal aorta (hereinafter, referred to as AA), is passed through a curved portion 100 of the CIA, and is further advanced inside the CIA toward the constricted part Le. The guide wire 1 is passed through the external iliac artery (hereinafter, referred to as EIA), the common femoral artery (hereinafter, referred to as CFA), the superficial femoral artery (hereinafter, referred to as SFA), and the popliteal artery (hereinafter, referred to as Pop.A), and reaches the anterior tibial artery (ATA). The guide wire 1 is indwelled near the constricted part Le, and the treatment of the constricted part Le is performed by inserting a combined instrument such as a catheter into the blood vessel along the guide wire 1. In this way, in the crossover method, because the guide wire is passed through the CIA, EIA, and CFA, which have a relatively large degree of curvature among the lower limb blood vessels, there is still room for improvement in the performance with respect to efficiently transmitting the operations performed by an operator of rotating the guide wire to the distal end of the guide wire (torque transmission performance). Furthermore, there is still room for improvement in the performance with respect to enabling a combined instrument such as a catheter, which is inserted into the blood vessel along the guide wire, to be passed through the CIA, which has a particularly large degree of curvature (delivery performance).

The disclosed embodiments have been made in order to solve the problems described above, and an object of the disclosed embodiments is to provide a guide wire used for treatment of a constricted part in the lower limb blood vessels, having both excellent torque transmission performance and delivery performance during treatment using the crossover method.

### SOLUTION TO PROBLEM

The disclosed embodiments have been made to solve at least a part of the problems described above, and can be realized as the following aspects.
(1) According to an aspect of the disclosed embodiments, a guide wire is provided. The guide wire comprises a core shaft, wherein the core shaft has a main body part, being a portion 350 mm or more and 750 mm or less from a distal end, and which is made of nickel-titanium alloy, and an outer diameter of the main body part is 0.58 mm or more and 0.73 mm or less.
   According to this configuration, because the main body part, which is a portion 350 mm or more and 750 mm or less from the distal end of the core shaft, is made of nickel-titanium alloy, and has an outer diameter of 0.58 mm or more and 0.73 mm or less, it is possible to provide a guide wire having both excellent torque transmission performance and delivery performance during treatment using the crossover method. Specifically, because the main body part is made of nickel-titanium alloy, even when the main body part is passed through the CIA, EIA, and CFA, which have a large degree of curvature, it is possible to reduce the deterioration in operability caused by deformation of the core shaft. Furthermore, because the outer diameter of the main body part is 0.58 mm or more, by placing the main body part near the CIA, it is possible to easily advance a combined instrument such as a catheter from one leg to the other leg along the guide wire. Moreover, because the main body part is 0.73 mm or less, by placing the main body part near the CIA, it is possible for the rotation operations made by the operator to be efficiently transmitted to the distal end of the guide wire. In addition, because the main body part is in the range of 350 mm or more and 750 mm or less from the distal end of the core shaft, it is possible to place the main body part near the CIA irrespective of the length of the blood vessels of the patient, and the torque transmission performance and the delivery performance of the guide wire can be improved in a greater number of patients.
(2) In the guide wire of the aspect above, in the core shaft, the outer diameter of the main body part may be 0.71 mm or less.

According to this configuration, because the outer diameter of the main body part, which is a portion 350 mm or more and 750 mm or less from the distal end of the core shaft, is 0.71 mm or less, it is possible to further improve the torque transmission performance during treatment using the crossover method.

Note that the disclosed embodiments may be implemented in various modes, such as a guide wire, a manufacturing method of a guide wire, a manufacturing method of a catheter, an endoscope, and a dilator.

### BRIEF DESCRIPTION OF DRAWINGS

Fig. 1 is an explanatory diagram illustrating an overall configuration of a guide wire according to a first embodiment.
Fig. 2 is an explanatory diagram illustrating a longitudinal cross-section of the entire guide wire according to the first embodiment.
Fig. 3 is an explanatory diagram illustrating an A1-A1 cross-section of the guide wire according to the first embodiment.
Fig. 4 is an explanatory diagram illustrating a B 1-B 1 cross-section of the guide wire according to the first embodiment.
Fig. 5 is an explanatory diagram illustrating a C1-C1 cross-section of the guide wire according to the first embodiment.
Fig. 6 is a diagram showing the test results of a torque transmission performance test.
Fig. 7 is an explanatory diagram showing the test method of a torque transmission performance test.
Fig. 8 is a diagram showing the test results of a delivery performance test.
Fig. 9 is an explanatory diagram showing the test method of a delivery performance test.
Fig. 10 is an explanatory diagram showing an example of good delivery performance.
Fig. 11 is an explanatory diagram showing an example of poor delivery performance.
Fig. 12 is a diagram showing measurement results of the bending load of a catheter.
Fig. 13 is a diagram illustrating a state in which a guide wire is indwelled in the lower limb blood vessels of a human body.
Fig. 14 is an explanatory diagram illustrating a longitudinal cross-section of the entire guide wire according to a second embodiment.
Fig. 15 is an explanatory diagram illustrating an A2-A2 cross-section of the guide wire according to the second embodiment.
Fig. 16 is an explanatory diagram illustrating a B2-B2 cross-section of the guide wire according to the second embodiment.
Fig. 17 is an explanatory diagram illustrating a C2-C2 cross-section of the guide wire according to the second embodiment.
Fig. 18 is an explanatory diagram illustrating a longitudinal cross-section of the entire guide wire according to a third embodiment.
Fig. 19 is an explanatory diagram illustrating an A3-A3 cross-section of the guide wire according to the third embodiment.
Fig. 20 is an explanatory diagram illustrating a B3-B3 cross-section of the guide wire according to the third embodiment.
Fig. 21 is an explanatory diagram illustrating a C3-C3 cross-section of the guide wire according to the third embodiment.
Fig. 22 is an explanatory diagram illustrating a longitudinal cross-section of the entire guide wire according to a fourth embodiment.
Fig. 23 is an explanatory diagram illustrating an A4-A4 cross-section of the guide wire according to the fourth embodiment.
Fig. 24 is an explanatory diagram illustrating a B4-B4 cross-section of the guide wire according to the fourth embodiment.
Fig. 25 is an explanatory diagram illustrating a C4-C4 cross-section of the guide wire according to the fourth embodiment.
Fig. 26 is an explanatory diagram illustrating the lower limb blood vessels of a human body.
Fig. 27 is an explanatory diagram showing enlarged the X region in Fig. 26.

### EMBODIMENTS OF THE INVENTION

A guide wire is a medical instrument that is inserted by a physician or the like into a blood vessel or digestive organ, and is used in treatment and examinations.

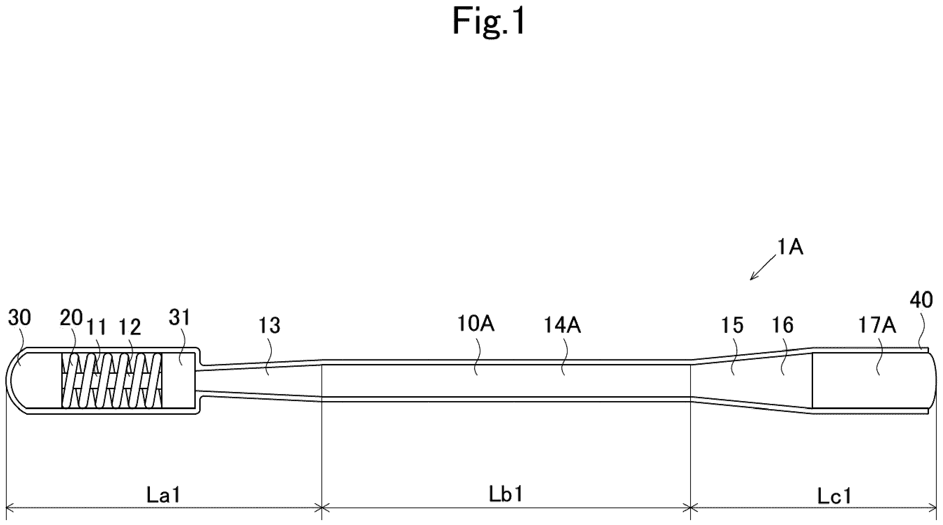

Hereinafter, the left side in each of Figs. 1, 2, 14, 18, and 22 is referred to as the "distal end side" of the guide wire of the disclosed embodiments and each constituent member of the guide wire, and the right side is referred to as the "rear end side" of the guide wire and each constituent member. The distal end side of the guide wire is the side that is inserted into the body first when the guide wire is inserted into the body, and the rear end side of the guide wire is the side (near side) that is operated by an operator such as a physician. Furthermore, the end portion located on the distal end side of the guide wire and each constituent member of the guide wire is described as the "distal end", and a portion including the "distal end" that extends partway from the distal end toward the rear end side is described as the "distal end portion". Similarly, the end portion located on the rear end side of the guide wire and each constituent member of the guide wire is described as the "rear end", and a portion including the "rear end" that extends partway from the rear end toward the distal end side is described as the "rear end portion".

The left-right direction in each of Figs. 1, 2, 14, 18, and 22 is referred to as the long axis direction of the guide wire and each constituent member of the guide wire. Furthermore, the direction orthogonal to the long axis direction is referred to as the radial direction of the guide wire and each constituent member of the guide wire.

For convenience of the description, each of Figs. 1 to 27 include portions where the guide wire and each constituent member of the guide wire are shown with a size whose relative ratio that is different from the actual relative ratio.

In the present application, the outer diameter of the core shaft is an average value of all of the outer diameters that have been measured. Furthermore, in the present application, the outer diameter of the core shaft being substantially constant means that the maximum value among the outer diameters of the core shaft is less than or equal to 1.05 times the minimum value. In the present application, the outer diameter of the core shaft is measured by an outer diameter measurement instrument that performs measurements in a non-contact mode using laser light, and is measured at intervals of 0.15 mm or less in the long axis direction of the core shaft.

### <First Embodiment>

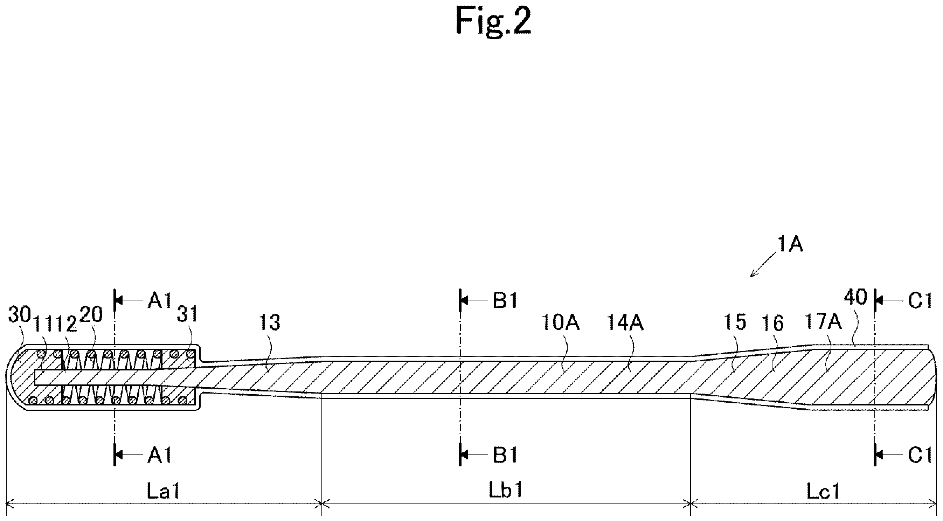

Fig. 1 is an explanatory diagram illustrating an overall configuration of a guide wire 1A according to a first embodiment. In Fig. 1, the inside of a resin film 40 is illustrated as seen through the resin film 40. Fig. 2 is an explanatory diagram illustrating a longitudinal cross-section of the entire guide wire 1A according to the first embodiment.

The guide wire 1A of the first embodiment is a medical instrument that is used by being inserted percutaneously into a blood vessel to treat a constricted part occurring in the lower limb blood vessels. The guide wire 1A includes a core shaft 10A, a coil 20 that covers a portion of the outer periphery of the core shaft 10A, and a resin film 40. The distal end portion of the coil 20 and the distal end portion of the core shaft 10A are fixed by a distal end side fixing portion 30. Furthermore, the rear end portion of the coil 20 and the core shaft 10A are fixed by a rear end side fixing portion 31.

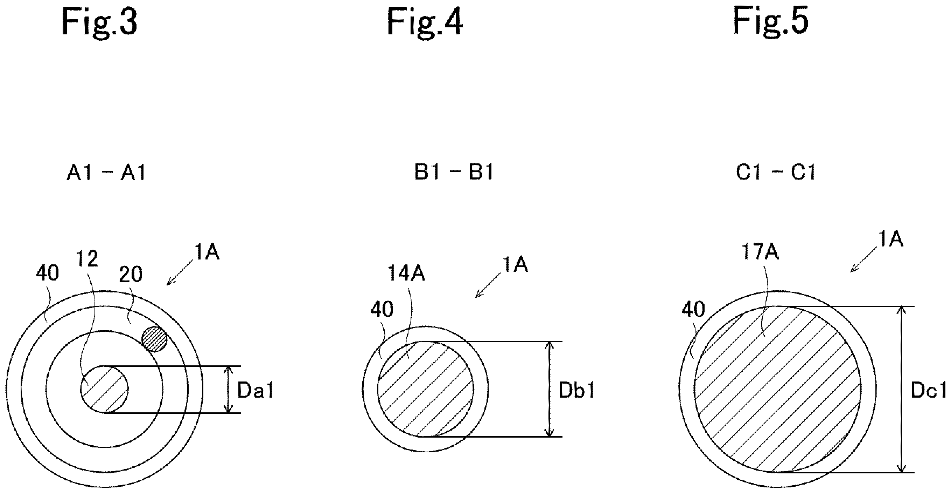

The core shaft 10A is a member having an overall length of approximately 2,000 mm to 4,000 mm. The transverse cross-section of the core shaft 10A is circular, and the maximum outer diameter is 0.58 mm or more and approximately 1.0 mm or less. The core shaft 10A has a small diameter portion 11, a main body part 14A, and a large diameter portion 15A from the distal end side toward the rear end side. The small diameter portion 11 is a portion having a smaller outer diameter than an outer diameter Db1 of the main body part 14A. The small diameter portion 11 has a distal end side straight portion 12 and a distal end side tapered portion 13. The distal end side straight portion 12 constitutes the distal end of the core shaft 10A, and the outer diameter Da1 (Fig. 3) is substantially constant along the long axis direction of the core shaft 10A. The distal end side tapered portion 13 is provided between the distal end side straight portion 12 and the main body part 14A, and has a tapered shape in which the outer diameter gradually increases toward the rear end side of the core shaft 10A. A portion of the outer periphery of the small diameter portion 11 is covered by the coil 20. The portion of the small diameter portion 11 that is covered by the coil 20 is a reinforced portion. The main body part 14A is provided between the small diameter portion 11 and the large diameter portion 15 of the core shaft 10A, and the outer diameter Db1 (Fig. 4) is substantially constant along the long axis direction of the core shaft 10A. The large diameter portion 15 is a portion having a larger outer diameter than an outer diameter Db1 of the main body part 14A. The large diameter portion 15 has a rear end side tapered portion 16 and a rear end side straight portion 17A. The rear end side tapered portion 16 is provided between the main body part 14A and the rear end side straight portion 17A, and has a tapered shape in which the outer diameter gradually increases toward the rear end side of the core shaft 10A. The rear end side straight portion 17A constitutes the rear end of the core shaft 10A, and the outer diameter Dc1 (Fig. 5) is substantially constant along the long axis direction of the core shaft 10A. The large diameter portion 15 functions as a high-rigidity portion having a higher torsional rigidity than the main body part 14A.

In the guide wire 1A of the first embodiment, La1 is the length of the small diameter portion 11 in the long axis direction. Lb1 is the length of the main body part 14A in the long axis direction. Lc1 is the length of the large diameter portion 15 in the long axis direction.

The main body part 14A is made of nickel-titanium alloy. A nickel-titanium alloy is an alloy having a composition consisting primarily of nickel and titanium. For example, a nickel-titanium alloy is an alloy composed of approximately 54 to 57.0 wt% of nickel, and a remaining portion composed of titanium. Furthermore, a nickel-titanium alloy can sometimes contain inclusions such as carbon, cobalt, copper, or chromium. The portions of the core shaft 10A other than the main body part 14A can be made of a material such as nickel-titanium alloy, stainless alloy (such as SUS302, SUS304, or SUS316), piano wire, nickel-chromium alloy, cobalt alloy, or tungsten. In the present embodiment, the main body part 14A and the portions of the core shaft 10A other than the main body part 14A are made of the same material, that is, a nickel-titanium alloy.

The coil 20 is an example of a reinforcing body described in the claims of the present application. The coil 20 is a tubular member that covers a portion of the outer periphery of the small diameter portion 11. The coil 20 is formed by winding a narrow-diameter metallic element wire in a spiral shape. The coil 20 can be made of a material such as nickel-titanium alloy, stainless alloy (such as SUS302, SUS304, or SUS316), piano wire, nickel-chromium alloy, cobalt alloy, or tungsten.

The distal end portion of the core shaft 10A and the distal end portion of the coil 20 are fixed by the distal end side fixing portion 30. The rear end portion of the core shaft 10A and the rear end portion of the coil 20 are fixed by the rear end side fixing portion 31. The distal end portion of the distal end side fixing portion 30 is formed in a hemispherical shape. The distal end side fixing portion 30 and the rear end side fixing portion 31 are formed, for example by a metal solder such as silver solder or gold folder, or by an adhesive using an epoxy resin or the like.

The resin film 40 is a thin film member that covers the entire length of the outer periphery of the guide wire 1A. The resin film 40 is formed of, for example, polyvinyl alcohol, polyvinylpyrrolidone, polyethylene glycol, polyacrylamide, polyacrylic acid, sodium polyacrylate, polyurethane, polytetrafluoroethylene, perfluoroalkoxyalkane, poly(2-hydroxyethyl methacrylate), maleic anhydride-based copolymer, ethylene vinyl alcohol copolymer, 2-methacryloyloxyethyl phosphorylcholine or a copolymer thereof, (2-hydroxyethyl methacrylate)-styrene block copolymer, various synthetic polypeptides, collagen, hyaluronic acid, cellulosic polymer, and mixtures of these.

Fig. 3 is an explanatory diagram illustrating an A1-A1 cross-section of the guide wire 1A according to the first embodiment. Fig. 3 shows a transverse cross-section of the distal end side straight portion 12. Fig. 4 is an explanatory diagram illustrating a B1-B1 cross-section of the guide wire 1A according to the first embodiment. Fig. 4 shows a transverse cross-section of the main body part 14A. Fig. 5 is an explanatory diagram illustrating a C1-C1 cross-section of the guide wire 1A according to the first embodiment. Fig. 5 shows a transverse cross-section of the rear end side straight portion 17A.

The transverse cross-section of the distal end side straight portion 12 has a circular shape with an outer diameter Da1. The distal end side straight portion 12 is the thinnest portion of the core shaft 10A. The outer diameter Da1 of the distal end side straight portion 12 is smaller than the outer diameter Db1 of the main body part 14A. The transverse cross-section of the main body part 14A has a circular shape with an outer diameter Db1. The outer diameter Db1 of the main body part 14A is larger than the outer diameter Da1 of the distal end side straight portion 12 and smaller than the outer diameter Dc1 of the rear end side straight portion 17A. The transverse cross-section of the rear end side straight portion 17A has a circular shape with an outer diameter Dc1. The rear end side straight portion 17A is the thickest portion of the core shaft 10A. The outer diameter Dc 1 of the rear end side straight portion 17A is larger than the outer diameter Db1 of the main body part 14A.

As described below, in consideration of the torque transmission performance and the delivery performance of the guide wire used in the crossover method, the outer diameter Db1 of the main body part 14A is 0.58 mm or more and 0.73 mm or less. Furthermore, as described below, the outer diameter Db1 of the main body part 14A is more preferably 0.58 mm or more and 0.71 mm or less.

### <Torque Transmission Performance Test>

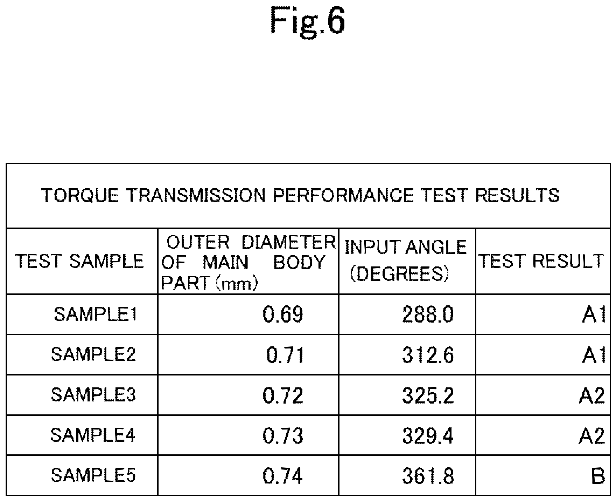

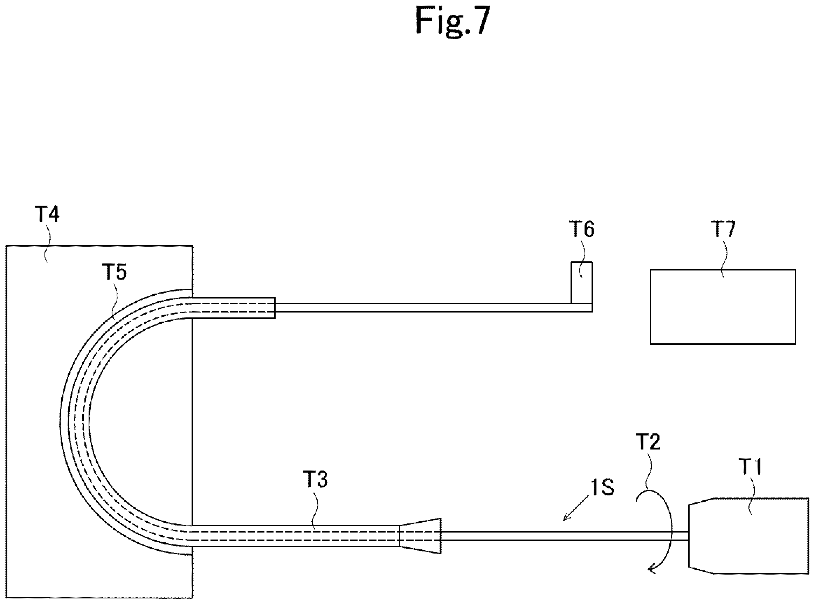

Fig. 6 is a diagram showing the test results of a torque transmission performance test. In the torque transmission performance test, as shown in Fig. 6, five types of guide wires (samples 1 to 5) were prepared, each having a different outer diameter Db1 of the main body part. As shown in Fig. 6, a larger sample number indicates a larger outer diameter Db1 of the main body part 14A. The test method of the torque transmission performance test will be described below using Fig. 7. The "input angle" in Fig. 6 is an evaluation value of each sample obtained by the torque transmission performance test, and the "test result" is a result determined from the evaluation value of the "input angle". Here, for reasons described below, samples in which the input angle was 315 degrees or less were rated "A1", samples greater than 315 degrees and 360 degrees or less were rated "A2", and samples greater than 360 degrees were rated "B".

Fig. 7 is an explanatory diagram showing the test method of a torque transmission performance test. A torque performance test blood vessel model T4 is provided with a simulated blood vessel T5 that simulates the CIA and the blood vessels in the vicinity thereof. The torque transmission performance test is performed by the following procedure using the torque transmission performance test blood vessel model T4. First, a catheter T3 is disposed over the entire length of the simulated blood vessel T5. Then, one of the guide wires 1S among the samples 1 to 5 is inserted inside the catheter T3 such that the main body part is disposed over the entire length of the torque transmission performance test blood vessel model T4. Next, the rear end portion of the guide wire 1S is connected to a guide wire rotation unit T1, and is rotated by the rotation unit T1 in the direction of a rotation direction T2 in Fig. 7. A measurement marker T6 attached to the distal end portion of the guide wire 1S is imaged by a camera T7, and the input angle of the rotation unit T1 when the rotation angle becomes 180 degrees is recorded. The "input angle" column in Fig. 6 indicates, for each of the samples 1 to 5, the input angle of the rotation unit T1 when the rotation angle of the measurement marker T6 becomes 180 degrees. The rotation angle of the measurement marker T6 corresponds to the rotation angle of the distal end portion of the guide wire 1S. The input angle of the rotation unit T1 corresponds to the rotation angle when the operator rotates the rear end portion of the guide wire 1S.

In the test results of the present test, it was determined that the samples having a test result of A1 and A2 had good torque transmission performance. In particular, it was determined that samples having a test result of A1 had superior performance. These criteria were determined in consideration of the fact that, when the operator rotates the distal end of the guide wire to determine the advancing direction at a branching part of the blood vessel or the like, the torque transmission performance is required to be at a level such that the distal end portion of the guide wire makes a half turn from a single finger operation by the operator (an operation that causes the rear end portion of the guide wire to rotate by approximately a single turn). A sample exhibiting superior torque transmission performance such that the operator can cause the distal end portion of the guide wire to make a half turn without performing a single turn of the rear end portion of the guide wire was rated A1, and a sample in which the distal end portion of the guide wire can at least make a half turn with a single turn or less was rated A2. From the test results, it can be determined that sample 3 and sample 4 had good torque transmission performance, and sample 1 and sample 2 had superior torque transmission performance. From this, it can be determined that the torque transmission performance is good when the outer diameter Db1 of the main body part 14A is 0.73 mm or less, and the torque transmission performance is superior when the outer diameter Db1 of the main body part 14A is 0.71 mm or less.

### <Delivery Performance Test>

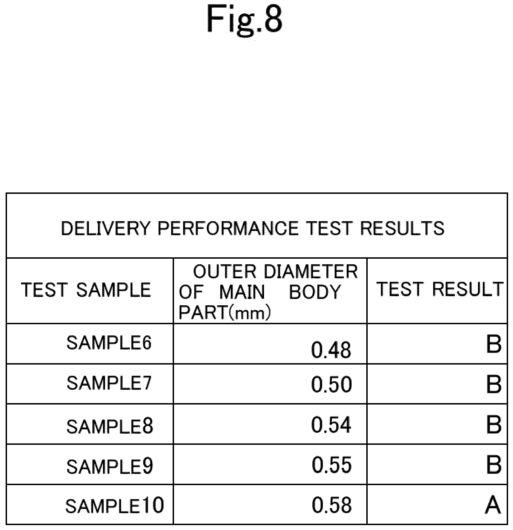

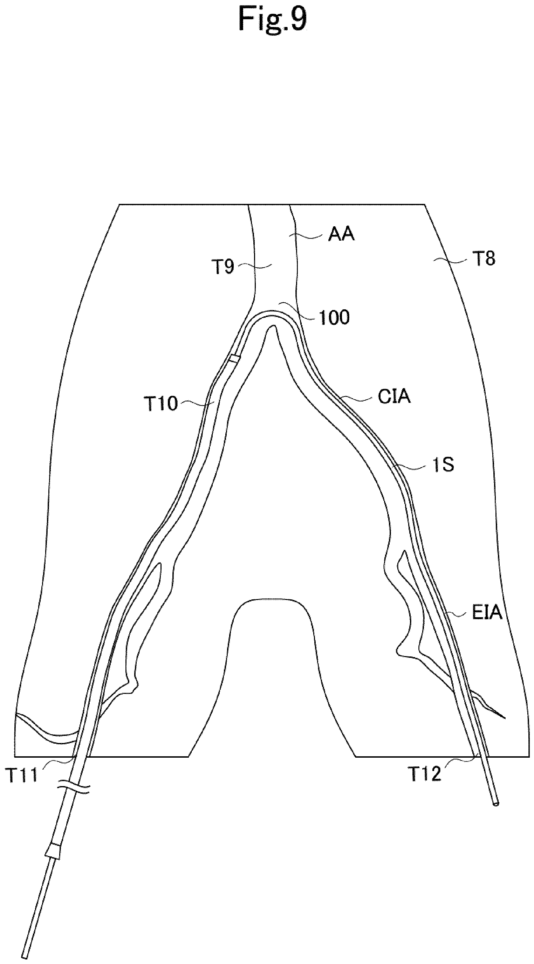

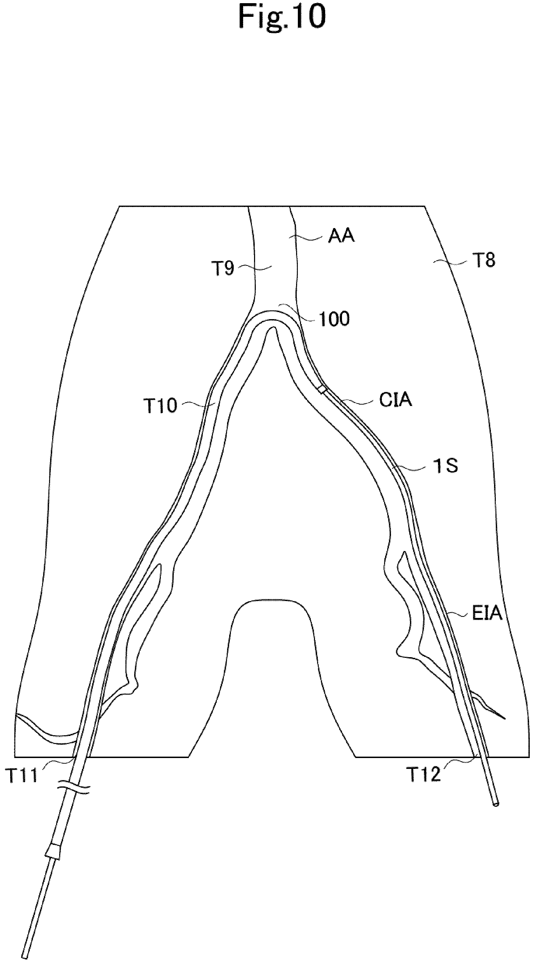

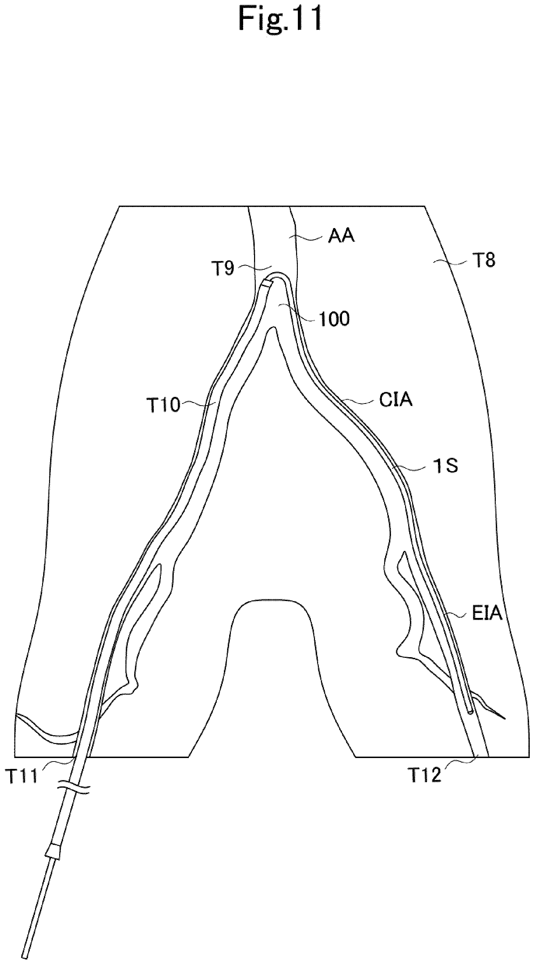

Fig. 8 is a diagram showing the test results of a delivery performance test. In the delivery performance test, as shown in Fig. 8, five types of guide wires (samples 6 to 10) were prepared, each having a different outer diameter Db1 of the main body part. As shown in Fig. 8, a larger sample number indicates a larger outer diameter Db1 of the main body part 14A. The test method and the evaluation method of the delivery performance test will be described below using Figs. 9 to 11. The "test result" in Fig. 8 is the result obtained by evaluation using the evaluation method described below. Here, samples in the state shown in Fig. 10 at the time of delivery were rated "A", and samples in the state shown in Fig. 11 were rated "B". The samples having the test result A showed good delivery performance. The samples having the test result B showed poor delivery performance.

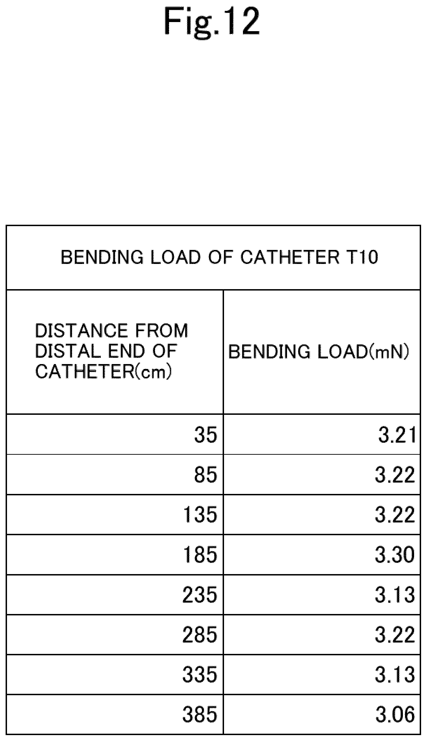

Fig. 9 is an explanatory diagram showing the test method of a delivery performance test. Fig. 10 is an explanatory diagram showing an example of good delivery performance in the delivery performance test. Fig. 11 is an explanatory diagram showing an example of poor delivery performance in the delivery performance test. Fig. 12 illustrates measurement results of the bending load of a catheter used in the delivery performance test.

A delivery performance test blood vessel model T8 is provided with a simulated blood vessel T9 that simulates the shape of the CIA and blood vessels near the CIA. The delivery performance test is performed by the following procedure. First, one of the guide wires 1S among the samples 6 to 10 is inserted into the simulated blood vessel T9 from an opening portion T11 provided at an end portion of the simulated blood vessel T9, and is indwelled in a state where the main body part is disposed in the curved portion 100 of the CIA. Then, a catheter T10 is inserted into the simulated blood vessel T9 from the opening portion T11 along the guide wire 1A. In the present test, a DESTINATION (registered trademark) manufactured by Terumo Corp., which is a catheter that is generally used in lower limb blood vessel treatment, was used as the catheter T10. The catheter T10 had an inner diameter of approximately 2.24 mm, and an outer diameter of approximately 2.79 mm. Fig. 12 shows bending loads, which correspond to the distance from the distal end of the catheter T10. Next, the catheter T10 is advanced beyond the curved portion 100 of the CIA toward the EIA. At this time, as shown in Fig. 10, the samples in which the position of the main body part of the indwelled guide wire 1A did not significantly change due to moving the catheter T10, and enabled the catheter T10 to be advanced beyond the curved portion 100 of the CIA were determined to have good delivery performance. On the other hand, as shown in Fig. 11, the samples in which the position of the main body part of the indwelled guide wire 1S significantly changed due to moving the catheter T10 toward the EIA, and did not allow the catheter T10 to be advanced beyond the curved portion 100 of the CIA due to the main body part and the catheter T10 being pushed out toward the AA were determined to have poor delivery performance.

In the test results of the present test shown in Fig. 8, sample 10, which enabled the catheter T10 to be advanced beyond the curved portion 100 of the CIA, was determined to have good delivery performance, and samples 6 to 9, which did not allow the catheter T10 to be advanced beyond the curved portion 100 of the CIA, were determined to have poor delivery performance. As a result, it can be determined that good delivery performance is obtained when the outer diameter Db1 of the main body part 14A is 0.58 mm or more.

### <Position and Area Evaluation of Main Body Part>

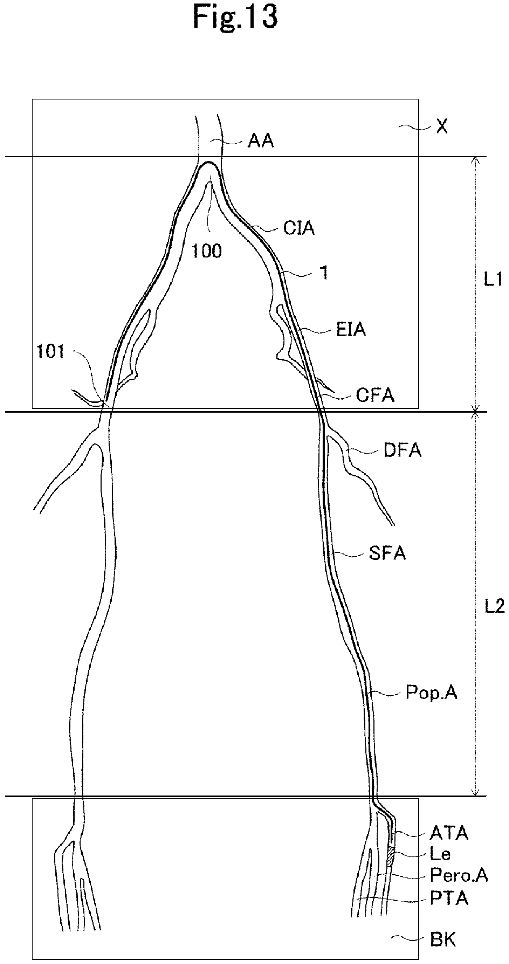

Fig. 13 is an explanatory diagram illustrating a state in which the guide wire 1A is indwelled in the lower limb blood vessels of a human body by the crossover method. Here, the position and area of the main body part for positioning, in a curved portion of the lower limb blood vessels, the main body part having the outer diameter Db1 obtained from the torque transmission performance test and the delivery performance test described above when the guide wire is indwelled in the lower limb blood vessel by the crossover method will be investigated. As shown in Fig. 13, the lower limb blood vessels are formed having the AA (abdominal aorta), the CIA (common iliac artery), EIA (external iliac artery), and the CFA (common femoral artery) in this order from the abdomen toward the feet. The CFA branches into the SFA (superficial femoral artery) and the DFA (deep femoral artery), the SFA connects to the Pop.A (popliteal artery), and the Pop.A branches into the ATA (anterior tibial artery), the PTA (posterior tibial artery), and the Pero.A (peroneal artery). The BK region in Fig. 13 represents the lower limb blood vessels including the ATA, the PTA, and the Pero.A. In the lower limb blood vessels, blood vessels having a large degree of curvature such as the curved portion 100 of the CIA tend to frequently occur in the CIA, the EIA, and the CFA. The X region shown in Fig. 13 represents a region containing blood vessels having a large degree of curvature. L1 shown in Fig. 13 is the length from the end of the AA to the end of the CFA. According to CT scan data obtained from patients, L1 is approximately 150 mm. Furthermore, because the punctured portion 101, which is the position in which the guide wire 1A is inserted in the crossover method, is often near the end of the CFA, the distance from the punctured portion 101 to the end of the AA is substantially equal to L1. As a result, the length from the punctured portion 101 to the end of the CFA of the other leg is approximately 300 mm. Moreover, L2 is the length from the end of the CFA to the end of the Pop.A. According to CT scan data obtained from patients, L2 is approximately 350 mm to approximately 450 mm. For example, when a constricted part Le occurs in the ATA, PTA, or Pero.A, and the length of L2 is approximately 350 mm when the distal end portion of the guide wire 1A is disposed in these blood vessels, the area of the core shaft 10A of the guide wire 1A that is approximately 350 mm or more and approximately 650 mm or less from the distal end is disposed in the X region. Furthermore, when the length of L2 is approximately 450 mm or more, the area of the core shaft 10A of the guide wire 1A that is approximately 450 mm or more and approximately 750 mm or less from the distal end is disposed in the X region. As a result, when a constricted part Le that has occurred in the BK region is treated by the crossover method, it is highly likely that the area of the core shaft 10A of the guide wire 1A that is approximately 350 mm or more and approximately 750 mm or less from the distal end is disposed in the X region.

The portion of the core shaft 10A that is disposed in the X region changes depending on the location in which the constricted part Le occurs and the location of the punctured portion 101. The inventors identified a position in the human body in which the punctured portion 101 is easily set, and made the settings described such that the main body part 14A is positioned in the X region even when the guide wire is indwelled in a position such that the distal end of the guide wire reaches the BK region, in which a constricted part Le is likely to occur and the torquability and deliverability are most required.

As described above, in the guide wire 1A of the present embodiment, the portion of the core shaft 10A that is 350 mm or more and 750 mm or less from the distal end is the main body part 14A. With such a configuration, when a constricted part Le that has occurred in the BK region or the like is treated by the crossover method, the main body part 14A is more likely to be disposed in the X region regardless of the length of L2. The outer diameter Db1 of the main body part 14A is 0.73 mm or less. As a result, when the main body part 14A is inserted into the curved portion of a blood vessel, the amount of strain caused by bending deformation is reduced, and in addition, by reducing the contact resistance the main body part 14A receives from the inner wall of the blood vessel, the guide wire 1A can exhibit good torque transmission performance, even in the crossover method. Furthermore, the outer diameter Db1 of the main body part 14A may be 0.71 mm or less. As a result, when the main body part 14A is inserted into the curved portion of a blood vessel, the amount of strain caused by bending deformation is further reduced, and in addition, by further reducing the contact resistance the main body part 14A receives from the inner wall of the blood vessel, the guide wire 1A can exhibit superior torque transmission performance, even in the crossover method. Moreover, because the outer diameter Db 1 of the main body part 14A is 0.58 mm or more, the guide wire 1A can exhibit good delivery performance, even in the crossover method.

The main body part 14A is made of nickel-titanium alloy. As a result, even when the main body part is inserted into the CIA, EIA, and CFA, which have a relatively large degree of curvature, it is possible to reduce the deterioration in operability caused by deformation of the core shaft 10A.

In the guide wire 1A of the first embodiment, the outer diameter Db1 of the main body part 14A is substantially constant over the length of the long axis direction. As a result, for example, compared to a case where the outer diameter Db1 of the main body part 14A is not substantially constant, such as a tapered shape, in which the outer diameter Db1 of the main body part 14A gradually increases along the long axis direction, or a stepped shape, in which the outer diameter Db1 of the main body part 14A increases at regular intervals in the long axis direction, good torque transmission performance and delivery performance can be exhibited regardless of the relative positional relationship between the main body part 14A and the blood vessel. In other words, even when the distal end side, or the rear end side, of the main body part 14A is disposed in the CIA, EIA, and CFA, which have a relatively large degree of curvature, good torque transmission performance and delivery performance can be exhibited in either case.

The guide wire 1A of the first embodiment includes the resin film 40. As a result, the sliding resistance between the outer peripheral surface of the guide wire 1A and the inside of the blood vessel can be reduced, and the slidability of the guide wire 1A inside the blood vessel improves.

The guide wire 1A of the first embodiment includes the coil 20. As a result, the strength of the reinforced portion, being the portion of the guide wire 1A that is inserted into narrow-diameter blood vessels further toward the end, can be improved by the reinforcing body. In the first embodiment, the reinforced portion is a portion of the small diameter portion 11 whose outer periphery is covered by the coil 20. Furthermore, in the guide wire 1A, the flexibility of the distal end side of the guide wire 1A can be improved by including the small diameter portion 11. Moreover, the guide wire 1A includes the distal end side tapered portion 13. As a result, the flexural rigidity of the core shaft 10A can be gradually increased from the distal end side straight portion 11 toward the main body part 14A, and because the stress is concentrated at a portion between the distal end side straight portion 12 and the main body part 14A, it is possible to reduce the likelihood that the core shaft 10A will become kinked. In addition, the guide wire 1A includes the large diameter portion 15. As a result, because the torsional rigidity of the rear end portion of the guide wire 1A is high, it is possible to further improve the torque transmission performance. Moreover, the guide wire 1A includes the rear end side tapered portion 16. As a result, the flexural rigidity of the core shaft 10A can be gradually increased from the main body part 14A toward the rear end side straight portion 17A, and because the stress is concentrated at a portion between the main body part 14A and the rear end side straight portion 17A, it is possible to reduce the likelihood that the core shaft 10A will become kinked.

### <Second Embodiment>

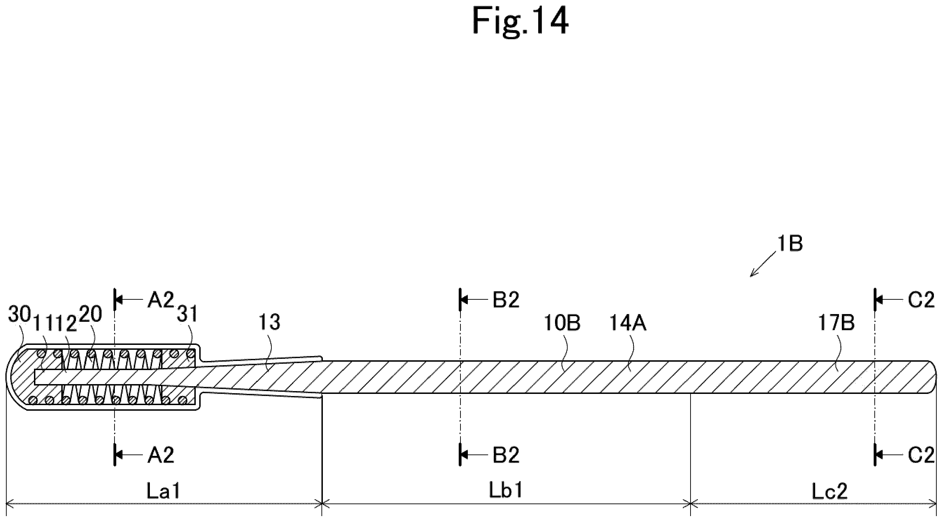

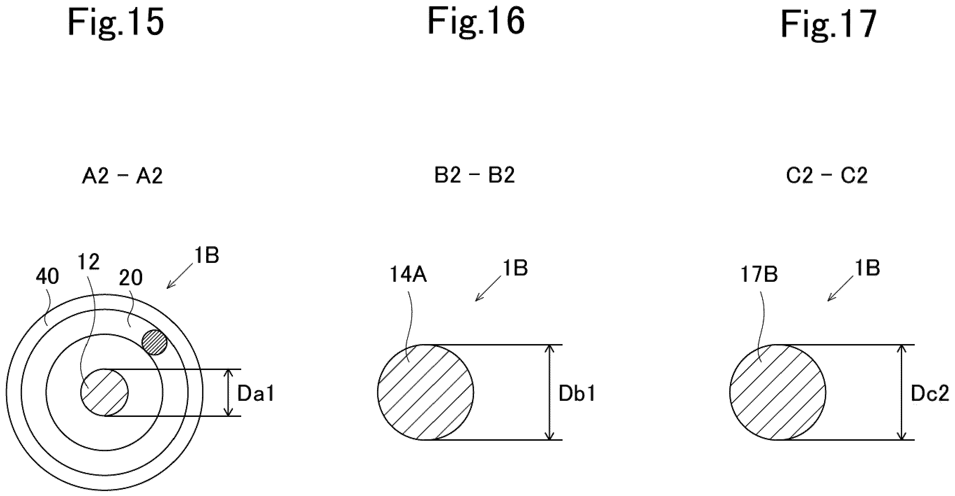

Fig. 14 is an explanatory diagram illustrating a longitudinal cross-section of the entire guide wire 1B according to a second embodiment. Fig. 15 is an explanatory diagram illustrating an A2-A2 cross-section of the guide wire 1B according to the second embodiment. Fig. 16 is an explanatory diagram illustrating a B2-B2 cross-section of the guide wire 1B according to the second embodiment. Fig. 17 is an explanatory diagram illustrating a C2-C2 cross-section of the guide wire 1B according to the second embodiment.

The guide wire 1B of the second embodiment differs from the guide wire 1A of the first embodiment in that it does not include the large diameter portion 15 (Fig. 2). The portions of the guide wire 1B of the second embodiment other than the large diameter portion 15 are the same as the guide wire 1A of the first embodiment. The guide wire 1B includes a core shaft 10B having a rear end side straight portion 17B. The rear end side straight portion 17B is further toward the rear end side than the main body part 14A, and is a portion that constitutes the rear end portion of the core shaft 10B. Lc2 is the length of the rear end side straight portion 17B in the long axis direction. The outer diameter Dc2 of the rear end side straight portion 17B is substantially equal to the outer diameter Db 1 of the main body part 14A.

The guide wire 1B of the second embodiment described above is capable of exhibiting effects that are equivalent to those of the guide wire 1A of the first embodiment. Because the outer diameter Dc2 of the rear end side straight portion 17B is substantially equal to the outer diameter Db 1 of the main body part 14A, for example, good torque transmission performance and delivery performance can be exhibited even when the CIA, EIA, and CFA, which have a large degree of curvature, are longer than expected, and the core shaft 10B further toward the rear end side than the main body part 14A is disposed inside these blood vessels.

### <Third Embodiment>

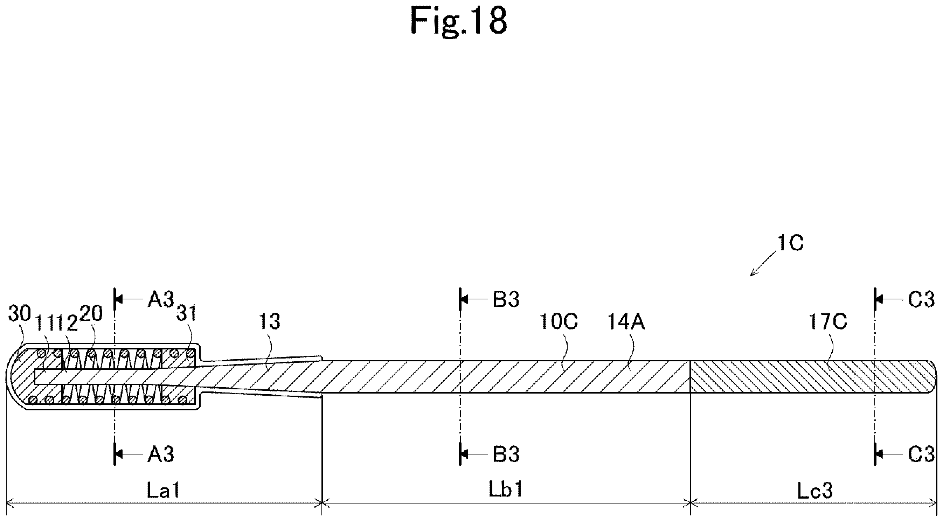

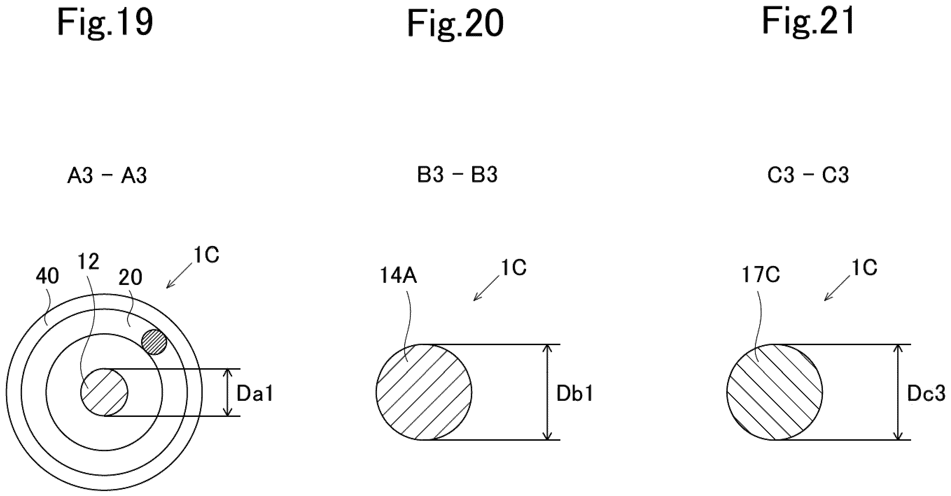

Fig. 18 is an explanatory diagram illustrating a longitudinal cross-section of the entire guide wire 1C according to a third embodiment. Fig. 19 is an explanatory diagram illustrating an A3-A3 cross-section of the guide wire 1C according to the third embodiment. Fig. 20 is an explanatory diagram illustrating a B3-B3 cross-section of the guide wire 1C according to the third embodiment. Fig. 21 is an explanatory diagram illustrating a C3-C3 cross-section of the guide wire 1C according to the third embodiment.

The guide wire 1A of the first embodiment and the guide wire 1C of the third embodiment are different in that the rear end side straight portion 17C of the guide wire 1C is made of a different material having a higher torsional rigidity than that of the main body part 14A. The portions of the guide wire 1C of the third embodiment other than the rear end side straight portion 17C are the same as the guide wire 1A of the first embodiment. In Fig. 18, the rear end side straight portion 17C is the portion depicted by a hatching having a different pattern to the hatching of the main body part 14A. The guide wire 1C includes a core shaft 10C having the rear end side straight portion 17C. The rear end side straight portion 17C is further toward the rear end side than the main body part 14A, and is a portion that constitutes the rear end portion of the core shaft 10C. The outer diameter Dc3 of the rear end side straight portion 17C is substantially equal to the outer diameter Db1 of the main body part 14A. Lc3 is the length of the rear end side straight portion 17C in the long axis direction. The rear end side straight portion 17C is configured by a different material having a transverse elastic modulus that is higher than that of the material of the main body part 14A. The rear end side straight portion 17C can be made of a material such as stainless alloy (such as SUS302, SUS304, or SUS316), piano wire, nickel-chromium alloy, cobalt alloy, or tungsten. The rear end portion of the main body part 14A and the distal end portion of the rear end side straight portion 17C are joined by a method such as soldering, an adhesive, or by welding.

The guide wire 1C of the third embodiment described above is capable of exhibiting effects that are equivalent to those of the guide wire 1A of the first embodiment. Furthermore, because the transverse elastic modulus of the material of the rear end side straight portion 17C is higher than the transverse elastic modulus of the material of the main body part 14A, the torsional rigidity of the rear end portion of the core shaft 10C is higher, which enables superior torque transmission performance to be exhibited.

### <Fourth Embodiment>

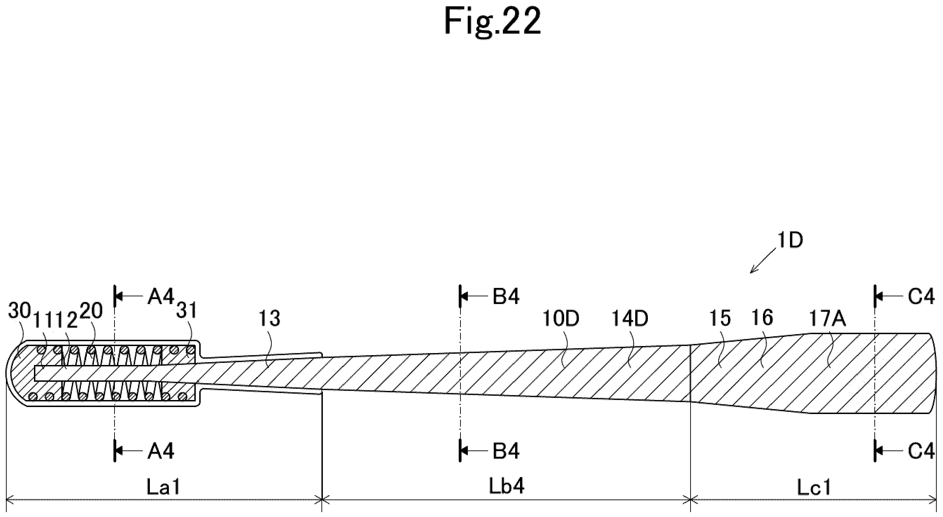

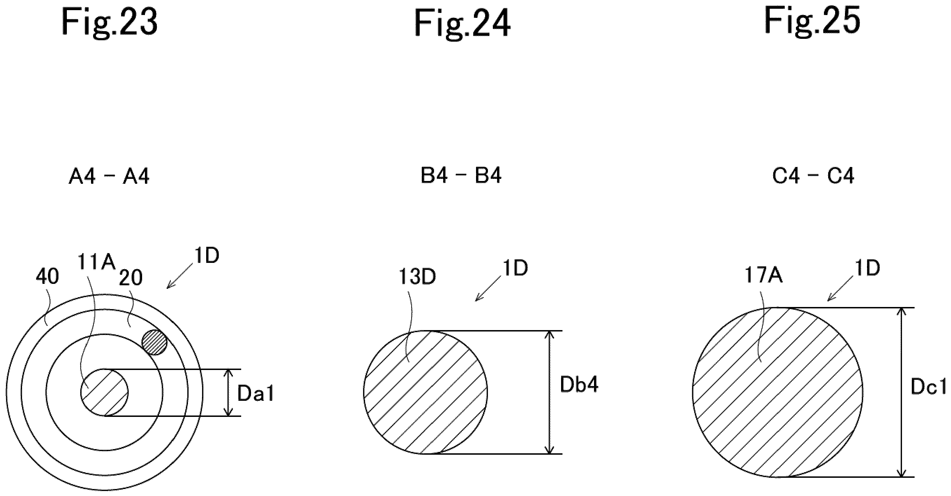

Fig. 22 is an explanatory diagram illustrating a longitudinal cross-section of the entire guide wire 1D according to a fourth embodiment. Fig. 23 is an explanatory diagram illustrating an A4-A4 cross-section of the guide wire 1D according to the fourth embodiment. Fig. 24 is an explanatory diagram illustrating a B4-B4 cross-section of the guide wire 1D according to the fourth embodiment. Fig. 25 is an explanatory diagram illustrating a C4-C4 cross-section of the guide wire 1D according to the fourth embodiment.

The guide wire 1A of the first embodiment and the guide wire 1D of the fourth embodiment are different in that the main body part 14D of the guide wire 1D has a tapered shape in which the outer diameter Db4 gradually increases toward the rear end side of the core shaft 10D. The portions of the guide wire 1D of the fourth embodiment other than the main body part 14D are the same as the guide wire 1A of the first embodiment. The guide wire 1D includes a core shaft 10D having the main body part 14D. The outer diameter Db4 of the main body part 14D gradually increases toward the rear end side of the core shaft 10D. Lb4 is the length of the main body part 14D in the long axis direction.

The guide wire 1D of the fourth embodiment described above is capable of exhibiting effects that are equivalent to those of the guide wire 1A of the first embodiment. In the guide wire 1D, because the main body part 14D has a tapered shape, it is possible for the flexural rigidity of the core shaft 10D to be gradually increased from the small diameter portion 11 toward the large diameter portion 15. As a result, the stress is concentrated at the main body part 14D, and it is possible to reduce the likelihood that the main body part 14D will become kinked.

### <Modification 1>

The guide wire 1A of the first embodiment was described such that the distal end side straight portion 12, the main body part 14A, and the rear end side straight portion 17A have a straight shape, and the distal end side tapered portion 13 and the rear end side tapered portion 16 have a diameter that increases from the distal end side toward the proximal end side. However, the outer diameter of each portion of the core shaft may be a straight shape, in which the outer diameter is substantially constant in the long axis direction, or a tapered shape, in which the outer diameter gradually increases or decreases toward the rear end side of the core shaft. Alternatively, the outer diameter of each portion of the core shaft may be a stepped shape, in which the outer diameter increases or decreases at regular intervals in the long axis direction of the core shaft.

### <Modification 2>

The transverse cross-section of the core shaft 10 of the guide wire 1A of the first embodiment is circular. However, the transverse cross-section of the core shaft 10 does not have to be circular, and may be a rectangular shape, such as a square shape or rectangle shape, or a triangular shape. In particular, when the transverse cross-section of the distal end side straight portion 12 of the guide wire 1A has a shape such as rectangle shape that is anisotropic in a direction that is easily deformed, it becomes easier for the operator to shape the distal end portion of the guide wire.

### <Modification 3>

In the guide wire 1A of the first embodiment, the torsional rigidity of the rear end side is higher than that of the distal end side. However, the core shaft may have a configuration in which the torsional rigidity of the distal end side in the long axis direction is higher. For example, the torsional rigidity of the small diameter portion may be higher than the torsional rigidity of the main body part.

### <Modification 4>

The resin film 40 of the first embodiment is made of a single type of resin, in which the characteristics do not change. However, the characteristics of the resin film that covers the outer periphery of the core shaft may change in the long axis direction of the core shaft. For example, the main body part and the large diameter portion may be covered with a hydrophobic resin film, or only the large diameter portion may be covered with a hydrophobic resin film. A guide wire in which the large diameter portion is covered with a hydrophobic resin film can be provided with the sliding performance of the guide wire, while also having a frictional force to an extent that enables the operator to easily grip the guide wire. Furthermore, the entire main body part may be covered by a resin film having the same characteristics, or different portions of the main body part may be covered by a plurality of resin films having different characteristics. Moreover, part or all of the core shaft may be covered such that a plurality of resins form a layered structure. For example, the coil and the outer periphery of the main body part may be covered by a resin film containing urethane, and the outer periphery of the resin film containing urethane may be further covered by a hydrophilic resin film.

### <Modification 5>

The reinforcing body of the first embodiment is formed by winding a metallic element wire having a narrow diameter in a spiral shape. However, the reinforcing body may be a hollow coil formed by twisting together a plurality of metallic element wires. Furthermore, the guide wire does not have to include the reinforcing body. When the reinforcing body is not included, the outer periphery of the distal end portion of the core shaft may be covered by a resin film.

### DESCRIPTION OF REFERENCE NUMERALS

1, 1A, 1B, 1C, 1D, 1S Guide wire
10A, 10B, 10C, 10D Core shaft
11 Small diameter portion
12 Distal end side straight portion
13 Distal end side tapered portion
14A, 14D Main body part
15 Large diameter portion
16 Rear end side tapered portion
17A, 17B, 17C Rear end side straight portion
20 Coil
30 Distal end side fixing portion
31 Rear end side fixing portion
40 Resin film
100 Curved portion of CIA
101 Punctured portion
La1 Length of core shaft small diameter portion
Lb1, Lb4 Length of core shaft main body part
Lc1, Lc2, Lc3 Length of core shaft large diameter portion
Da1 Outer diameter of distal end side straight portion
Db1, Db4 Outer diameter of main body part
Dc1, Dc2, Dc3 Outer diameter of rear end side straight portion
AA Abdominal aorta
CIA Common iliac artery
EIA External iliac artery
CFA Common femoral artery
SFA Superficial femoral artery
DFA Deep femoral artery
Pop.A Popliteal artery
ATA Anterior tibial artery
PTA Posterior tibial artery
Pero.A Peroneal artery
BK Below-knee region
Le Blood vessel constricted part
L1 Length from curved portion of AA to end of CFA
L2 Length from end of CFA to end of Pop.A
T1 Guide wire rotation unit
T2 Rotation direction of guide wire
T3, T10 Catheter
T4 Torque performance test blood vessel model
T5 Simulated blood vessel
T6 Measurement marker
T7 Camera
T8 Delivery performance test blood vessel model
T9 Simulated blood vessel
T11, T12 Opening portion

## Claims

1. A guide wire comprising a core shaft, wherein
the core shaft has a main body part, being a portion 350 mm or more and 750 mm or less from a distal end, and which is made of nickel-titanium alloy, and an outer diameter of the main body part is 0.58 mm or more and 0.73 mm or less.

2. The guide wire according to claim 1, wherein
in the core shaft, an outer diameter of the main body part is 0.58 mm or more and 0.71 mm or less.
